# EUROPÄISCHE PATENTANMELDUNG

(11) **EP 1 327 871 A1**
(43) Veröffentlichungstag der Anmeldung: **16.07.2003**
(21) Anmeldenummer: 02028916.1
(22) Anmeldetag: 23.12.2002
(51) Int. Cl.: G01M 17/00, G01M 19/00, G01N 33/00

(54) **Verfahren und Vorrichtung zur Detektion eines Geruches**

(30) Priorität: 11.01.2002 DE 10200735
(71) Anmelder: ROBERT BOSCH GMBH, 70442 Stuttgart (DE)
(72) Erfinder: Ingrisch, Kurt, 72762 Reutlingen (DE); Bauer, Michael, 72076 Tuebingen (DE); Elbe, Dieter, 74343 Sachsenheim (DE); Brinz, Thomas, 73266 Bissingen Unter Der Teck (DE); Krummel, Christian, 72138 Kirchentellinsfurt (DE)

(57) **Zusammenfassung**

Es wird ein Verfahren zur Detektion eines Geruches eines Stoffgemisches mit mindestens einer geruchsaktiven Komponente vorgeschlagen, wobei eine der Komponenten des Stoffgemisches als Leitsubstanz ausgewählt, die Konzentration der Leitsubstanz mittels eines für die Leitsubstanz spezifischen Sensors (4) ermittelt und ein Schaltelement (2) in Abhängigkeit von der Konzentration der Leitsubstanz betätigt wird (Figur 1).

## Beschreibung

### Stand der Technik

Die Erfindung geht von einem Verfahren zur Detektion eines Geruches eines Stoffgemisches gemäß der im Oberbegriff des Patentanspruches 1 näher definierten Art sowie von einer Vorrichtung zur Detektion eines Geruches eines Stoffgemisches gemäß der im Oberbegriff des Patentanspruches 14 näher definierten Art aus.

Zur spezifischen Erfassung von Geruchssituationen ist ein Verfahren bekannt, bei dem eine sogenannte "elektronische Nase" eingesetzt wird. Hierbei werden mehrere Sensoren einer Geruchssituation ausgesetzt. Mittels der Sensoren ermittelte Sensorsignale werden dann durch eine spezifische Signalverarbeitung ausgewertet, um so die Geruchssituation zu charakterisieren. Die Signalverarbeitung erfolgt mittels einer Hauptkomponentenanalyse, mittels neuronaler Netze oder dergleichen. Die Bestimmung der Geruchssituation erfolgt ohne Kenntnis spezifischer geruchsaktiver Komponenten, die ein die Geruchssituation auslösendes Stoffgemisch umfaßt. Es ist daher bei dem bekannten Verfahren nicht bekannt, ob der oder die eingesetzten Sensoren auf eine geruchsaktive Komponente der Geruchssituation oder auf ein Korrelat reagieren, das in anderen Stoffen ebenfalls vorkommen kann, welche auch geruchsfrei sein können.

Im Bereich der Automobiltechnik werden zur Zeit zwei chemische Substanzklassen zur automatischen Lüftungsklappensteuerung herangezogen. Bei diesen Substanzklassen handelt es sich einerseits um reduzierende und andererseits um oxidierende Gase. Die Substanzklassen zeichnen sich jeweils durch eine Leitsubstanz aus. Bei reduzierenden Gasen ist die Leitsubstanz in der Regel durch Kohlenmonoxid und bei oxidierenden Gasen ist die Leitsubstanz in der Regel durch Stickoxid gebildet. Die beiden Substanzklassen repräsentieren zwei Schaltsituationen. Bei der einen sollen Abgase einer Otto-Brennkraftmaschine, bei der Kohlenmonoxid anfällt, bei der anderen sollen Abgase einer Diesel-Brennkraftmaschine, bei der Stickoxide anfallen, detektiert werden.

Zur Detektion einer Substanzklasse wird hierzu in der Regel ein Metalloxid-Halbleiter-Gassensor eingesetzt. Es kann aber auch ein Sensor für beide Substanzklassen eingesetzt werden. Bisher eingesetzte Sensoren sind häufig unspezifisch, da sie neben einem jeweils ausgewählten Zielgas, beispielsweise Kohlenmonoxid, auch andere Gase derselben Substanzklasse detektieren. Folglich kann mit einem Kohlenmonoxid-Sensor auch ein anderes Gas, beispielsweise Ammoniak, detektiert werden, um eine damit verbundene Geruchssituation durch Betätigung einer Lüftungsklappe oder eines Aktivkohlefilters aus einem Innenraum eines Kraftfahrzeuges fernzuhalten.

Die Empfindlichkeiten bekannter Sensoren korrelieren nicht mit Geruchsschwellen, die den jeweils geruchsaktiven Komponenten eines Stoffgemisches zu eigen sind. Daher muß ein bekannter Sensor in der Praxis auf eine bestimmte Schaltsituation eingestellt werden. Dies hat zur Folge, daß Geruchssituationen, bei denen der Sensor nur ein schwaches Signal liefert, nicht erfaßt werden, oder daß Geruchssituationen, bei denen der Sensor ein hohes Meßsignal liefert, überbewertet werden. Insbesondere letzteres ist aus sicherheitstechnischen Gründen und aus Gründen der Standzeit eines mittels des Sensors gesteuerten Schaltelements, wie einer Lüftungsklappe, nicht wünschenswert.

### Vorteile der Erfindung

Das Verfahren zur Detektion eines Geruches eines Stoffgemisches mit den Merkmalen nach dem Oberbegriff des Patentanspruches 1, bei welchem Verfahren eine der Komponenten des Stoffgemisches als Leitsubstanz ausgewählt, die Konzentration der Leitsubstanz mittels eines für die Leitsubstanz spezifischen Sensors ermittelt und ein Schaltelement in Abhängigkeit von der Konzentration der Leitsubstanz betätigt wird, hat den Vorteil, daß ein spezifischer Geruch eines mindestens eine geruchsaktive Komponente umfassenden Stoffgemisches durch zuvorige Auswahl einer der Komponenten als Leitsubstanz und eine geeignete Auslegung des Sensors bezüglich der Leitsubstanz zuverlässig erkannt werden kann. Dadurch wird die Möglichkeit geboten, durch Auswahl einer geeigneten Leitsubstanz/Sensor-Paarung eine Geruchssituation durch Detektion einer einzigen Komponente eines für die Geruchssituation verantwortlichen Stoff- bzw. Gasgemisches sowie dessen Nachweisgrenze genau zu spezifizieren. Ferner ist ein Abgleich der Empfindlichkeit des Sensors sowie eine Prüfbarkeit des Sensors bei dessen Fertigung möglich, da hierzu beispielsweise ein synthetisch erzeugter Standard herangezogen werden kann, dessen chemische Eigenschaften denjenigen der ausgewählten Leitsubstanz entsprechen.

Zweckmäßig wird das Schaltelement bei Überschreiten eines voreingestellten Konzentrationsschwellenwertes betätigt. Der Konzentrationsschwellenwert muß natürlich oberhalb der Nachweisgrenze der als Leitsubstanz ausgewählten Komponente liegen, sollte aber so gewählt sein, daß die menschliche Nase den Geruch des betreffenden Stoffgemischs noch nicht wahrnehmen kann.

Das Verfahren nach der Erfindung ist insbesondere zum Einsatz bei einem Lüftungssystem eines Kraftfahrzeuges geeignet, wobei dann das Schaltelement eine Lüftungsklappe, wie eine Umluftklappe eines Lüftungssystems, oder auch eine entsprechende Klappe einer Klimaanlage sein kann. Die jeweilige Klappe wird bei Überschreiten des voreingestellten Konzentrationsschwellenwertes geschlossen.

Das Verfahren nach der Erfindung kann zur Detektion verschiedenster Gerüche eingesetzt werden. So kann beispielsweise der Geruch eines Skunks, Güllegeruch, Stallgeruch, Kanalisationsgeruch, ein bei der Teerverarbeitung auftretender Geruch, Brandgeruch oder auch Benzingeruch detektiert werden. All diese Gerüche sind durch ein Stoffgemisch aus mehreren geruchsaktiven Komponenten bedingt. So sind beispielsweise dem Mephitinae- bzw. Skunk-Geruch folgende geruchsaktiven Komponenten zuzurechnen:
- trans-2-Buten-1-thiol
- trans-2-Butenylthioacetat
- 3-Methyl-1-butanthiol
- 2-Methyl-2-propanthiol
- 3-Methylbutanylthioacetat
- 2-Methylchinolin
- 2-Chinolinmethylthioacetat
- 2-Chinolinnmethanthiol
- 2-Phenylethanthiol
- 2-Phenylmethanthiol
- bis-[trans-2-butenyl]-disulfid
- trans-2-butenyl-3-methylbutyl-disulfid
- bis-(3-methylbutyl)-disulfid

Als Leitsubstanz dieses den Skunk-Geruch bildenden Gas- bzw. Stoffgemischs wird vorzugsweise 3-Methyl-1-Buthanthiol ausgewählt. Der Sensor ist hinsichtlich dieser geruchsaktiven Komponente auszulegen.

Einem Güllegeruch sind hingegen die in der nachfolgend dargestellten Tabelle aufgeführten, geruchsaktiven Komponenten zuzurechnen. In der Tabelle sind die geruchsaktiven Komponenten teilweise hinsichtlich ihrer Konzentration, ihrer Toxizität, ihrer Nachweisgrenze, ihrer Grenze hinsichtlich der Wahrnehmung durch die menschliche Nase sowie der Art des jeweiligen Geruches dargestellt.

**Tabelle:**

| **Bezeichnung** | **Ca. Konz. (%)** | **Toxizität** | **Nachweisgren- ze (ppb)** | **Wahrneh- mungsgren- ze (ppb)** | **Beschreibung** |
|---|---|---|---|---|---|
| Propionsäure | 0,71 | - | 3,6 | 300 | |
| Dimethylsulfid | 0,19 | + | 1,1 | 1,1 | Verdorbenes Gemüse |
| Iso-Valeriansäure | 0,03 | 2g/kg LD50 | 1,2 | | |
| Essigsäure | 0,16 | 10ppm | 10,2 | 1 | Essig |
| Indol | 0,01 | | 1 | | Fäkalien |
| 3-Methyl-1H-Indol (Skatol) | 0,02 | | 1,2 | 470 | Fäkalien, stechend |
| p-Cresol | 0,05 | 5ppm | 8 | | |
| Acetylaldehyd | 0,50 | 100ppm | 210 | 210 | Fruchtig, beißend |
| Phenol | 0,01 | 10ppm | 5,7 | 1 | Medizinisch |
| Valeriansäure | 0,02 | ++ | 20 | 20 | |
| Wasserstoffsulfid | | | 0,5 | 4,7 | Verfaulte Eier |
| Methylmercaptan | | | 0,5 | 2,1 | Verdorbener Kohl |
| Buttersäure | | 2,9g/kg LD50 | 1,1 | 1 | Verdorbenes Fleisch |
| Azeton | | | 4 | 100 | Süß, beißend |
| Diethylsulfid | | | 6 | 6 | Verdorbenes Gemüse |
| Dimethylamin | | | 37 | 37 | Fisch, beißend |
| Dimethylsulfoxid | | | | | |
| Hexansäure | | 3g/kg LD50 | | | |
| Thiocyansäure-Methylester | | | | | |
| Formaldehyd | | | | 1 | Stroh, beißend |
| Methylethylketon | | | | 10 | Süß |
| Methylamin | | | | 2,1 | Fisch, beißend |
| Diethylamin | | | | 500 | Fisch, beißend |

Zur Durchführung des Verfahrens nach der Erfindung wird aus den in der vorstehenden Tabelle aufgeführten, geruchsaktiven Komponenten vorzugsweise Propionsäure, Acetylaldehyd, Dimethylamin oder Buttersäure als Leitsubstanz ausgewählt.

Der bei dem Verfahren nach der Erfindung eingesetzte Sensor kann bei der Herstellung und/oder auch nachträglich mittels eines synthetischen, der Leitsubstanz entsprechenden Standards kalibriert und/oder geprüft werden. Dadurch kann eine zuverlässige Funktion des jeweils eingesetzten Sensors gewährleistet werden.

Eine kostengünstige Durchführung des Verfahrens nach der Erfindung wird ermöglicht, wenn der für die Leitsubstanz spezifische Sensor von einem Halbleiter-Gassensor mit einer sensitiven Schicht gebildet ist. Alternativ können natürlich auch andere Sensoren eingesetzt werden, beispielsweise ein Sensor, der nach einem Infrarotadsorptionsspektroskopie-Verfahren arbeitet, ein Chemolumineszenz-Detektor, ein Surface-Acoustic-Wave-Sensor oder ein Bulk-Acoustic-Wave-Sensor.

Bei Verwendung eines Halbleiter-Gassensors bei Durchführung des Verfahrens nach der Erfindung umfaßt das Sensorelement bevorzugt einen thermisch isolierten Membranbereich, eine Heizstruktur, eine Elektrodenstruktur sowie eine sensitive Schicht. Die sensitive Schicht ist auf die Leitsubstanz des Stoffgemisches ausgelegt. Bei der Herstellung des Gassensors besteht die sensitive Schicht aus pulverförmigen Metalloxiden, die mittels eines Brennprozesses versintert werden.

Die sensitive Schicht besteht beispielsweise aus einem Metalloxid der Metalle Zinn, Wolfram, Zink, Eisen, Molybdän und/oder Titan. Das Metalloxid kann mit einem Metall dotiert sein, und zwar mit Kupfer, Nickel, Molybdän, Renium, Zink, Chrom, Aluminium, Cer, Mangan, Titan, Nickel, Cobalt, Ruthenium, Iridium und/oder Eisen. Ferner kann die sensitive Schicht auch mit Edelmetallen, wie Silber, Gold, Platin, Rhodium, Iridium und/oder Palladium, beispielsweise im Bereich zwischen 0,001 und 1 % dotiert sein. Das in der Regel oxidische Grundmaterial der sensitiven Schicht sowie die Beimengungen, die je nach Dotierung oxidisch oder metallisch vorliegen können, werden vorteilhaft so gewählt, daß ein bei Detektion der als Leitsubstanz ausgewählten, vorzugsweise geruchsaktiven Komponente des Stoffgemisches auftretendes Meßsignal, welches eine Widerstands-, Impedanzoder Kapazitätsänderung der sensitiven Schicht darstellt, ausreichend hoch und hinreichend selektiv für die ausgewählte Leitsubstanz ist. Die Bestimmung der Zusammensetzung der sensitiven Schicht bzw. Funktionsschicht erfolgt zweckmäßig empirisch.

Wenn der Sensor zur Detektion einer der geruchsaktiven Komponenten des den Skunk-Geruch bildenden Stoffgemisches, d. h. beispielsweise zur Detektion von 3-Methyl-1-Buthantiol, ausgelegt ist, besteht die sensitive Schicht des Halbleiter-Gassensors bei einer bevorzugten Ausführungsform aus einem Zinnoxid, das mit 0,0001 bis 1 % Kupfer und mit 0,001 bis 1 % Silber und Gold dotiert ist.

Wenn zur Detektion eines Güllegeruchs Buttersäure als Leitsubstanz ausgewählt wird, so besteht die sensitive Schicht nach einer vorteilhaften Ausführungsform aus Zinnoxid, Wolframoxid oder vorzugsweise Titanoxid, mit Dotierungen aus Vanadium, Colbalt, Lanthan und/oder vorzugsweise Molybdän sowie Palladium, Rhenium, Osram, Ruthenium und/oder vorzugsweise Iridium.

Alternativ kann bei der Detektion eines Güllegeruchs als sensitive Schicht Zinnoxid, gegebenenfalls mit Dotierungen von Tantal eingesetzt werden. Wenn eine Carbonsäure oder Acetaldehyd als Leitsubstanz ausgewählt wird, ist die sensitive Schicht des weiteren mit Titan, Nickel, Kobalt, Vanadium, Cer und/oder Molybdän dotiert. Diese Elemente, die Oberflächendotierungen darstellen, liegen dann in oxidischer Form vor. Wenn ein Amin als Leitsubstanz ausgewählt wird, kann das im Volumen mit Tantal dotierte Zinnoxid mit einer Oberflächendotierung aus Chrom, Zink, Ruthenium, Iridium, Platin und/oder Eisen versehen sein. Chrom, Zink und Eisen liegen dabei in oxidischer Form vor, wohingegen Ruthenium, Iridium und Platin in metallischer Form vorliegen.

Die Erfindung hat auch eine Vorrichtung zur Detektion eines Geruches, insbesondere bei einem Kraftfahrzeug, zum Gegenstand, wobei die Vorrichtung ein Schaltelement, wie beispielsweise eine Lüftungsklappe eines Lüftungssystems, umfaßt. Die Vorrichtung ist durch einen Sensor gekennzeichnet, der bezüglich einer vorbestimmten Leitsubstanz selektiv ist, die aus mehreren Komponenten eines Stoffgemisches ausgewählt ist, sowie durch eine mit dem Sensor verbundene Steuereinheit, mittels der das Schaltelement betätigbar ist.

Weitere Vorteile und vorteilhafte Ausgestaltungen des Gegenstandes nach der Erfindung sind der Beschreibung, der Zeichnung und den Patentansprüchen entnehmbar.

### Zeichnung

Ein Ausführungsbeispiel der Vorrichtung nach der Erfindung ist in der Zeichnung schematisch vereinfacht dargestellt und wird in der nachfolgenden Beschreibung näher erläutert.
Es zeigen
Figur 1 eine Prinzipskizze einer Vorrichtung nach der Erfindung bei einem Kraftfahrzeug;
Figur 2 einen Querschnitt durch einen Gassensor zur Durchführung des Verfahrens nach der Erfindung;
Figur 3 eine Aufsicht des Gassensors nach Figur 2; und
Figur 4 eine Figur 3 entsprechende Ansicht des Gassensors nach Figur 2, jedoch ohne gassensitive Schicht.

### Beschreibung des Ausführungsbeispiels

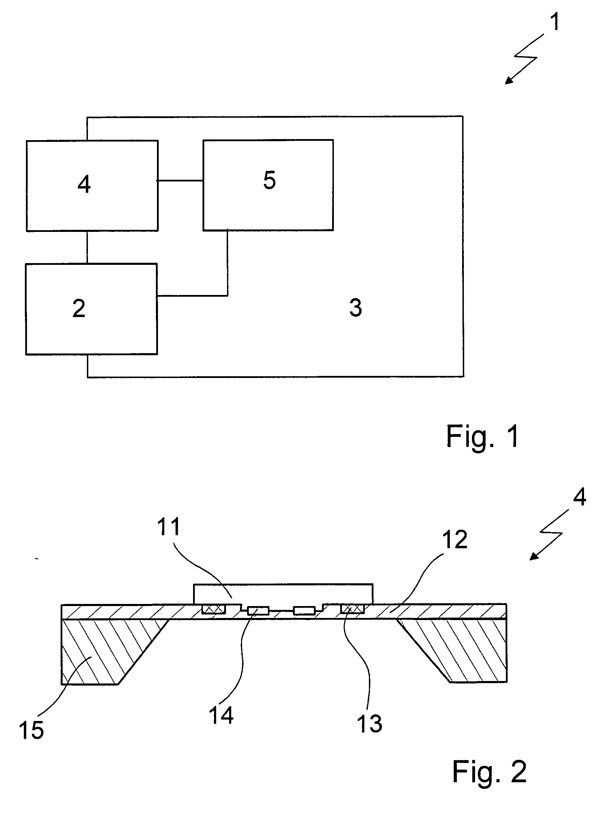

In Figur 1 ist eine Prinzipskizze eines Kraftfahrzeuges 1 dargestellt, welches mit einer erfindungsgemäßen Vorrichtung zur Detektion eines Geruches ausgestattet ist, so daß im Bedarfsfall eine Lüftungsklappe 2 geschlossen und ein Innenraum 3 des Kraftfahrzeuges 1 vor dem Eindringen des Geruches frühzeitig geschützt werden kann.

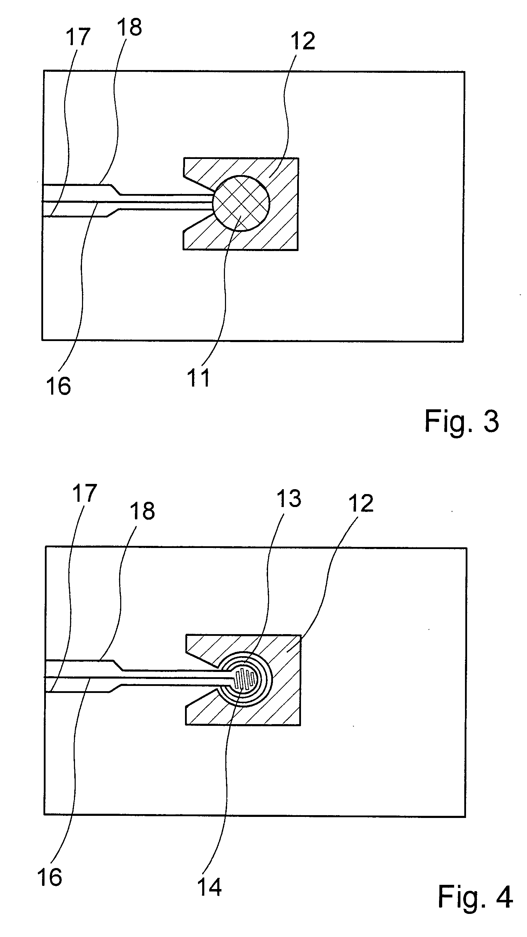

Die Vorrichtung zur Detektion des Geruches, beispielsweise eines Skunk-Geruches, umfaßt einen Sensor 4, der einen Halbleiter-Gassensor darstellt und in den Figuren 2 bis 4 näher dargestellt ist, sowie eine Steuereinheit 5, die einerseits mit dem Halbleiter-Gassensor 4 und andererseits mit der ein Schaltelement darstellenden Lüftungsklappe 2 verbunden ist.

Die Steuereinheit 5 dient zur Auswertung und Verarbeitung von mittels des Halbleiter-Gassensors 4 gewonnenen Meßsignalen, die mit einer Konzentration einer geruchsaktiven Komponente eines von einem Skunk abgegebenen, den Skunkgeruch bildenden Gas- bzw. Stoffgemisches korreliert sind. Die ausgewählte geruchsaktive Komponente bildet die sogenannte Leitsubstanz, auf die der Gassensor 4 ausgelegt ist. Im vorliegenden Fall ist als Leitsubstanz 3-Methyl-1-Buthantiol ausgewählt.

Der Gassensor 4 besteht aus einem Siliziumsubstrat 15, auf welchem eine Membran 12 angeordnet ist, in die wiederum eine Heizstruktur 13 sowie eine Elektrodenstruktur 14 eingebettet sind. Die Elektrodenstruktur 14 ist mit einer Zuleitung 16 und die Heizstruktur 13 ist mit Zuleitungen 17 und 18 zum Anschluß an eine Spannungsquelle versehen.

Die Heizstruktur 13 und die Elektrodenstruktur 14 sind von einer sensitiven Schicht 11 überdeckt, die aus Zinndioxid besteht und mit 0,1 % Kupfer sowie mit 0,1 % Silber und Gold dotiert ist.

Die Betriebstemperatur der sensitiven Schicht 11 liegt zwischen 100 und 400 °C. Diese Temperatur wird mittels der Heizstruktur 13 eingestellt.

Befindet sich in der Umgebung des Halbleiter-Gassensors 4 das zu detektierende Gas, d. h. in diesem Fall 3-Methyl-1-Buthantiol, so ändert sich der elektrische Widerstand der sensitiven Schicht 11, was mittels der Elektrodenstruktur 14 gemessen und mittels der Steuereinheit 5 ausgewertet wird. Alternativ zur Änderung des Widerstands der sensitiven Schicht 11 kann auch deren Impedanz- oder Kapazitätsänderung ausgewertet werden.

Die Steuereinheit 5 der Vorrichtung zur Detektion des Skunk-Geruches arbeitet derart, daß, wenn ein voreingestellter Schwellenwert hinsichtlich der Konzentration von 3-Methyl-1-Buthantiol, d. h. ein bestimmter Schwellenwert des Widerstands der sensitiven Schicht 11, erreicht ist, die Lüftungsklappe 2 geschlossen wird, so daß der Skunk-Geruch nicht in den Innenraum 3 des Kraftfahrzeugs 1 eindringen kann und Insassen des Kraftfahrzeuges 1 vor dem Skunk-Geruch geschützt sind.

Alternativ kann ein Gassensor der in den Figuren 2 bis 4 dargestellten Art auch zur Erfassung eines Gülle-Geruchs ausgelegt sein.

Der von Gülle abgegebene Geruch wird von einem Stoffgemisch gebildet, das als geruchsaktive Komponenten u. a. Buttersäure, Propionsäure, Acetylaldehyd und Dimethylamin umfaßt. Wählt man aus diesen geruchsaktiven Komponenten Buttersäure als Leitsubstanz aus, so ist es zweckmäßig, den in den Figuren 2 bis 4 dargestellten Gassensor mir einer sensitiven Schicht zu versehen, die aus Titandioxid besteht und mit Molybdän und Iridium dotiert ist.

## Patentansprüche

1. Verfahren zur Detektion eines Geruches eines Stoffgemisches mit mindestens einer geruchsaktiven Komponente, **dadurch gekennzeichnet, daß** mindestens eine der Komponenten des Stoffgemisches als Leitsubstanz ausgewählt und die Konzentration der Leitsubstanz mittels eines für die Leitsubstanz spezifischen Sensors (4) ermittelt wird.

2. Verfahren nach Anspruch 1, **dadurch gekennzeichnet, daß** ein Schaltelement (2) in Abhängigkeit von der Konzentration der Leitsubstanz betätigt wird.

3. Verfahren nach Anspruch 2, **dadurch gekennzeichnet, daß** das Schaltelement eine Lüftungsklappe (2) eines Kraftfahrzeuges ist.

4. Verfahren nach Anspruch 1, 2 oder 3, **dadurch gekennzeichnet, daß** die Leitsubstanz eine geruchsaktive Komponente eines von einem Skunk abgegebenen Stoffgemisches ist.

5. Verfahren nach Anspruch 4, **dadurch gekennzeichnet, daß** die Leitsubstanz des von einem Skunk abgegebenen Stoffgemisches 3-Methyl-1-Buthantiol ist.

6. Verfahren nach einem der Ansprüche 1 bis 5, **dadurch gekennzeichnet, daß** die Leitsubstanz eine geruchsaktive Komponente eines von Gülle abgegebenen Stoffgemisches ist.

7. Verfahren nach Anspruch 6, **dadurch gekennzeichnet, daß** die Leitsubstanz bei dem von Gülle abgegebenen Stoffgemisch Propionsäure, Acetylaldehyd, Dimethylamin oder Buttersäure ist.

8. Verfahren nach einem der Ansprüche 1 bis 7, **dadurch gekennzeichnet, daß** der für die Leitsubstanz spezifische Sensor mittels eines synthetischen, der Leitsubstanz entsprechenden Standards kalibriert und/oder geprüft wird.

9. Verfahren nach einem der Ansprüche 1 bis 8, **dadurch gekennzeichnet, daß** der für die Leitsubstanz spezifische Sensor ein Halbleiter-Gassensor (4) mit einer sensitiven Schicht (11) ist.

10. Verfahren nach Anspruch 9, **dadurch gekennzeichnet, daß** die sensitive Schicht (11) aus mindestens einem Metalloxid besteht.

11. Verfahren nach Anspruch 10, **dadurch gekennzeichnet, daß** das Metalloxid Zinndioxid, Wolframoxid oder Titandioxid ist.

12. Verfahren nach einem der Ansprüche 9 bis 11, **dadurch gekennzeichnet, daß** die sensitive Schicht (11) mit einem Metallion einer Konzentration zwischen 0,0001 und 1% dotiert ist.

13. Verfahren nach einem der Ansprüche 1 bis 12, **dadurch gekennzeichnet, daß** die sensitive Schicht (11) mit einem Edelmetall, wie Silber, Gold, Platin und/oder Palladium, im Bereich zwischen 0,001 und 1% dotiert ist.

14. Vorrichtung zur Detektion eines Geruches eines Stoffgemisches mit mindestens einer geruchsaktiven Komponente, insbesondere zum Einsatz bei einem Kraftfahrzeug, umfassend ein Schaltelement (2), **gekennzeichnet durch** einen Sensor (4), der bezüglich einer vorbestimmten Leitsubstanz selektiv ist, die aus den Komponenten des Stoffgemischs ausgewählt ist, sowie eine mit dem Sensor (4) verbundene Steuereinheit (5), mittels der das Schaltelement (2) betätigbar ist.

15. Vorrichtung nach Anspruch 14, **dadurch gekennzeichnet, daß** der Sensor ein Halbleiter-Gassensor (4) ist, der eine bezüglich der Leitsubstanz sensitive Schicht (11) hat.

16. Vorrichtung nach Anspruch 15, **dadurch gekennzeichnet, daß** die sensitive Schicht (11) aus einem vorzugsweise dotierten Metalloxid, wie Zinndioxid, Wolframoxid oder Titandioxid, gebildet ist.

17. Vorrichtung nach Anspruch 14 oder 15, **dadurch gekennzeichnet, daß** der Halbleiter-Gassensor (4) einen thermisch isolierten Membranbereich (12), eine Heizstruktur (13) und eine Elektrodenstruktur (14) umfaßt.
